# EUROPEAN PATENT SPECIFICATION

(11) **EP 2 876 604 B1**
(45) Date of publication and mention of the grant of the patent: **09.01.2019**
(21) Application number: 14194107.0
(22) Date of filing: 20.11.2014
(51) Int. Cl.: G06T 7/11, G06T 7/187, G06F 3/0484

(54) **Method of displaying an image based on pressure change, image processing apparatus**
Verfahren zum Anzeigen eines Bildes auf Basis von Druckänderung, Bildverarbeitungsvorrichtung
Procédé d'affichage d'image sur la base de changement de pression, appareil de traitement d'image

(30) Priority: 21.11.2013 KR 20130142285
(43) Date of publication of application: 27.05.2015
(73) Proprietor: Samsung Electronics Co., Ltd., Gyeonggi-do 443-742 (KR)
(72) Inventor: Lee, Yeon Ju, Gyeonggi-do (KR); Lee, Duhgoon, Gyeonggi-do (KR)
(74) Representative: Land, Addick Adrianus Gosling

(56) References cited:
- WO-A2-2006/013521
- US-B1- 8 286 102
- SIVEWRIGHT G J ET AL: "INTERACTIVE REGION AND VOLUME GROWING FOR SEGMENTING VOLUMES IN MR AND CT IMAGES", MEDICAL INFORMATICA, TAYLOR AND FRANCIS, BASINGSTOKE, GB, vol. 19, no. 1, 1 January 1994 (1994-01-01), pages 71-80, XP000603452, ISSN: 0307-7640

## Description

The disclosure relates to displaying images based on information such as a change in pressure. More particularly, the disclosure relates to a method of displaying an image and an image processing apparatus.

Image processing apparatuses provide an acquired image based on a variety of external information to a user. -A variety of external information is collected and then, converted into a predefined data format. An image is restored or generated based on the converted data of the collected external information, or by performing image processing on the restored image.

An image processing apparatus can be configured to collect various external information by use of, for example, visible light, infrared light, radiation, ultrasonic waves, or microwaves. In such cases, an image processing apparatus may be provided with one or more of a charge-coupled device, a transducer, a radiation detection panel, a radar detection panel, a radio frequency (RF) coil of a magnetic resonance apparatus, and the like in order to collect various external information.

Examples of the image processing apparatus may include infrared cameras, radiation imaging apparatuses, ultrasonic imaging apparatuses, magnetic resonance imaging apparatuses, radars, and the like.

SIVEWRIGHT, G.J. and ELLIOTT, P.J., in:"INTERACTIVE REGION AND VOLUME GROWING FOR SEGMENTING VOLUMES IN MR AND CT IMAGES", MEDICAL INFORMATICA, TAYLOR AND FRANCIS, BASINGSTOKE, GB, vol. 19, no. 1, 1 January 1994 (1994-01-01), pages 71-80, XP000603452, disclose a process of marking out selected parts of CT or MR images, wherein an algorithm detects regions of interest grown from a seed selected interactively by a user. The regions consist of connected areas or volumes having similar pixel intensity values. Threshold parameters for controlling the algorithm can be set interactively, or may be determined automatically form a sample region. Region growth can be successively extended into adjacent slices so building up a three-dimensional (3D) volume. WO 2006/013521 A2 discloses a data processing system comprising a pressure-sensitive input device for assigning a floating-point value to a parameter under control of a pressure applied to the device. The system is operative to detect a rate of change of the pressure to control the assigning, e.g., to validate the current value as being input or to unlock the value as set. Therefore, at least one aspect of the present invention is to provide an image processing apparatus according to claim 1, capable of controlling a displayed image based on pressure change, and a method of displaying an image according to claim 15.

The image processing apparatus allows to simply control an image displayed by a region growing technique without using a complicated manipulation method and a method of displaying an image. Region growing, for example, is a type of segmentation technique based on a similarity of adjacent pixels. First, a region growing can be started from a single pixel (that can be referred to as a "seed pixel"). The adjacent pixels are added then to the current region when there is a similarity to the region. Additional aspects of the invention will be set forth in part in the description which follows from the description, or may be learned by a person of ordinary skill in the art practicing the claimed invention.

The image processing apparatus includes a pressure sensor to sense an amount of applied pressure associated with a touch event, and an image processing controller configured to generate at least one image of an object by determining region growing parameters based on the sensed amount of pressure, and performing region growing from a reference location based on the determined region growing parameters.

For example, the region growing parameters may include at least one parameter selected from the group consisting of a range of a detection region of image data, a region growing rate, and a size of the object.

The image processing controller may increase a region growing rate, and/or the size of the object of at least one selected from the group consisting of the range of the detection region of the image data, as the sensed amount of pressure increases.

The image processing controller, which comprises hardware such as integrated circuitry configured for operation, may perform the region growing by comparing data of at least one location within the detection region with reference data of the reference location. The reference data may include at least one item_selected from the group consisting of image data of the reference location, location data of the reference location, and a shape of neighboring locations of the reference location. The image data of the reference location may include values acquired using the image data of the reference location and image data of the neighboring locations of the reference location.

The image processing controller typically includes or is integrated with a controller to receive signals output from, for example, a pressure sensor regarding detected pressure associated with a touch event, and may be configured to compare at least one portion of data of the detection region with the reference data of the reference location, and detect at least one portion of data based on the comparison result. The image processing controller may generate at least one image based on the detected image data when detection of image data is completed in the detection region.

The image processing controller may be configured generate a plurality of images of the object by performing region growing a number of times from different reference locations.

The image processing apparatus may further include a storage unit in which the plurality of generated images are sequentially stored in their order of generation. Other schemes to store the generated images may also be used.

The image processing apparatus may further include a display unit to display the at least one image.

The display unit may display a range of a detection region of image data, display at least one image among a plurality of sequentially stored images, or display an indicator indicating an amount of pressure sensed by the pressure sensor associated with a touch event.

The pressure sensor may be arranged with a touchscreen to sense the amount of pressure applied to the touchscreen and display an indicator indicating the sensed amount of pressure of the touch event.

The pressure sensor may be controlled by a controller that provides receive an instruction to select the reference location.

The method of displaying an image includes determining a reference location, determining parameters by sensing an amount of pressure and determining region growing parameters based on the sensed amount of pressure, and performing region growing from the determined reference location using the determined region growing parameters.

The region growing parameters may include at least one parameter selected from the group consisting of a range of a detection region of image data, a region growing rate, and a size of an object. The determining of the parameters may include increasing at least one parameter selected from the group consisting of the range of the detection region of image data, the region growing rate, and the size of the object as the sensed amount of pressure increases.

The performing of the region growing may include comparing data of at least one location within the detection region with reference data of the reference location, and performing the region growing according to the comparison result. The reference data may include at least one item selected from the group consisting of image data of the reference location, location data of the reference location, and a shape of neighboring locations of the reference location. The image data of the reference location may include values acquired using the image data of the reference location and image data of neighboring locations of the reference location.

The performing of the region growing may include comparing at least one portion of data of the detection region with the reference data of the reference location, and detecting the at least one portion of data based on the comparison result.

The performing of the region growing may further include generating at least one image based on the detected image data when the detecting of the image data from the detection region is completed.

The performing of the region growing may further include generating a plurality of images of an object by performing region growing a plurality of times from different reference locations.

The method may further include storing the plurality of generated images sequentially in the generation order, and displaying at least one image from among the plurality of sequentially stored images.

The method may further include displaying the at least one image by a display screen. The displaying of the at least one image may include displaying a range of a detection region of image data, displaying at least one image among a plurality of sequentially stored images, or displaying an indicator indicating an amount of pressure sensed by the pressure sensor.

The amount of pressure detected may be sensed by usage of a touchscreen.

The method may further include receiving an input instruction to select the reference location.

### BRIEF DESCRIPTION OF THE DRAWINGS

These and/or other aspects of the claimed invention will become more apparent and more readily appreciated from the following description of the embodiments, taken in conjunction with the accompanying drawings of which:
FIG. 1 is a block diagram illustrating one possible arrangement of an image processing apparatus according to an embodiment of the present invention;
FIG. 2 is a block diagram illustrating a pressure sensor according to an embodiment of the present invention:
FIG. 3 is a block diagram illustrating an image processing apparatus according to another embodiment of the present invention;
FIG. 4 is an exemplary diagram of an operation of an image processing controller;
FIG. 5 is an exemplary diagram illustrating an operation of an image processing controller;
FIG. 6 is an exemplary diagram illustrating an operation of an image processing controller;
FIG. 7 is an exemplary diagram illustrating an operation of an image processing controller;
FIG. 8 is an exemplary diagram illustrating an operation of an image processing controller;
FIG. 9 is an exemplary diagram illustrating an operation of an image processing controller;
FIG. 10 is an exemplary diagram illustrating an operation of an image processing controller;
FIG. 11 is an exemplary diagram illustrating an operation of an image processing controller;
FIG. 12 is an exemplary diagram illustrating an operation of an image processing controller;
FIG. 13 is an exemplary diagram illustrating an operation of an image processing controller;
FIG. 14 is an exemplary diagram illustrating an operation of an image processing controller;
FIGS. 15 and 16 are respective diagrams illustrating a region growing performed using an image processing apparatus;
FIGS. 17A, 17B and FIG. 17C are diagrams illustrating screens displaying indicators which indicate pressure;
FIGS. 18 and 19 are diagrams illustrating screens displayed by an image processing apparatus based on threshold change; and
FIGS. 20 and 21 are flowcharts illustrating methods of displaying an image.
FIG. 22 is an exemplary Radiation imaging apparatus.
FIG. 23 is an exemplary MRI apparatus.

### DETAILED DESCRIPTION

Reference will now be made in detail to certain aspects of the present invention, examples of which are illustrated in the accompanying drawings, wherein like reference numerals refer to like elements throughout.

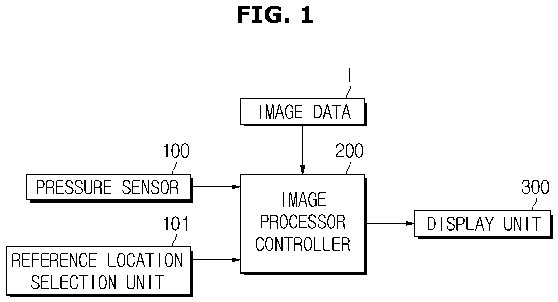

Image processing apparatuses according to embodiments of the present invention will be described with reference FIGS. 1 to 21.

FIG. 1 is a block diagram illustrating an image processing apparatus according to an embodiment of the present invention.

Referring now to FIG. 1, the image processing apparatus may include a pressure sensor 100, an image processing controller 200, and a display unit 300.

The pressure sensor 100 senses pressure applied (by a user), generates an electric signal in accordance with the sensed pressure, and transmits the electric signal to the image processing controller 200. In this regard, the envisioned user may include doctors, nurses, and medical radiation technologists who utilize image processing apparatuses or people who need to acquire images using image processing apparatuses. The pressure sensor 100 may also sense not just the presence of a certain amount of pressure (i.e. a predefined threshold) but also the amount of pressure, generate an electrical signal corresponding to the amount of pressure in accordance with the sensed amount of pressure, and transmit the electrical signal to the image processing controller 200.

The pressure sensor 100 may include one or more various pressure sensing devices to sense pressure. For example, the pressure sensing device may include a capacitor, an optical fiber, or a strain gauge. The pressure sensor 100 may sense pressure using the capacitor or the optical fiber or may sense pressure according to strain of the strain gauge. The pressure sensing device may also include a piezoelectric element or a pressure-sensitive film. Besides, the pressure sensor 100 may include various other types of pressure sensing devices used to sense pressure directly or indirectly.

The pressure sensor 100 may be installed so as to be coupled with various types of input units of the image processing apparatus. For example, the pressure sensor 100 may be installed in a keyboard device, a mouse device, a track ball device, or the like used to control the image processing apparatus.

The pressure sensor 100 may receive an instruction to select a reference location, if required.

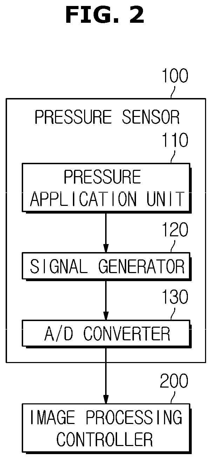

FIG. 2 is a block diagram illustrating a pressure sensor according to an embodiment of the present invention. According to an embodiment, the pressure sensor 100 may include a pressure application unit 110, a signal generator 120, and an analog/digital converter 130 as illustrated in FIG. 2. The logical arrangement shown is merely illustrative, and some of the items of the pressure sensor could be integrated physically or functionally.

The pressure application unit 110 of the pressure sensor 100 receives pressure applied from the outside. According to one embodiment, the pressure sensor can detect pressure directly applied thereto, and, for example, a user may directly apply pressure to the pressure sensor 100 using various devices for applying pressure. For example, the user may apply pressure to the pressure application unit 110 using a device such as a touch pen. The user may also directly apply pressure to the pressure application unit 110 using a finger or palm without using a device for applying pressure such as the touch pen.

When pressure is applied at the pressure application unit 110, the signal generator 120 generates an electric signal corresponding to an amount of the applied pressure and outputs the electric signal. In this case, the signal generator 120 may output an electric signal having frequency or amplitude which is increased correspondingly as pressure applied to the pressure application unit 110 increases.

When the signal output from the signal generator 120 comprises an analog signal, the analog/digital converter 130 may convert the analog signal to a digital signal. According to an embodiment, however, the analog signal output from the signal generator 120 may directly be transmitted to the image processing controller 200 without being converted into the digital signal. The electric signal output from the signal generator 120 or the analog/digital converter 130 may be transmitted to the image processing controller 200.

The image processing controller 200 may restore the image using image data "I "or may perform image processing. The image data I may include raw data collected by an image data collecting device of an imaging apparatus. The image data I may also include image data restored using raw data or image data processed by a predefined image processing method.

The image data I may be data acquired based on ultrasonic signals collected through a plurality of channels by transducers of an ultrasonic imaging apparatus. The image data I may also be data acquired based on radiation signals collected by a radiation detection panel of a radiation imaging apparatus. Here, the radiation imaging apparatus may include, for example, at least one radiation imaging apparatus capable of collecting information regarding the inner structure of the object via irradiation such as a digital radiography (DR) apparatus, a fluoroscopy apparatus, an electrocardiography apparatus, a computed tomography (CT) scanner, or a mammography system. The image data I may also be data acquired based on magnetic resonance signals collected by a radio frequency (RF) coil of a magnetic resonance imaging apparatus.

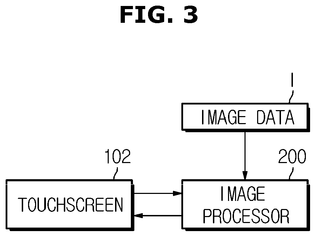

With continued reference to FIG. 2 and 3, Image data I may constitute an image. For example, the image data I may have pixel values corresponding to each of the pixels constituting the image. Particularly, the image data I may have a brightness or a color value of each of the pixels. The image formed using the image data I may be a two-dimensional image or a three-dimensional image. The image data I may be analog signals or digital signals according to devices used to collect the image data I. In addition, the image data I may be data acquired by previously applying a filter thereto.

The image processing controller 200, contains circuitry configured to perform a region growing operation from a predefined position selected in the image (reference location). Region growing is an image segmentation method by sequentially combining regions having the same or similar properties. In this regard, the properties may include various values of image data such as brightness and chroma.

The image processing controller 200 can be configured to determine region growing parameters, and then perform region growing based on the determined region growing parameters. The region growing parameters may include location of image data to be compared with reference data during the region growing process, a region growing rate, or a size of an object shown in the image. Here, the size of the object may include, for example, a thickness or length of blood vessel, as non-limiting possibilities.

The region growing parameters may be determined according to the amount of pressure sensed by the pressure sensor 100. For example, the range of the detection region of the image data may be used as the region growing parameter. In this case, as the amount of pressure sensed by the pressure sensor 100 increases, the range of the detection region of the image data to be compared with the reference data may be increased. As the amount of pressure sensed by the pressure sensor 100 decreases, the range of the detection region of the image data to be compared with the reference data may be decreased. For example, the region growing rate may also be used as the region growing parameter. In this case, as the amount of pressure sensed by the pressure sensor 100 increases, the region growing rate may be increased. As the amount of pressure sensed by the pressure sensor 100 decreases, the region growing rate may be decreased. For example, the size of the object may also be used as the region growing parameter. In this case, as the amount of pressure sensed by the pressure sensor 100 increases, a relatively larger object such as main artery may be detected in the image. As the amount of pressure sensed by the pressure sensor 100 decreases, a relatively smaller object such as a capillary may be detected.

The image processing controller 200 may detect a part of image data from the image data constituting the image to perform region growing. The image processing controller 200 may detect a part of image data from the image data of the detection region that is a portion of the entire image, and may perform a region growing therefrom.

In more detail, the image processing controller 200 may compare the image data I constituting the image with reference data, detect at least one portion of image data from the entire image data or a part of image data I based on the comparison result, and perform region growing. The image processing controller 200 may compare the entire image data I constituting the image with the reference data or may compare a part of image data I with the reference data.

The reference data may refer to data compared with image data of the detection region of the image. The reference data may include data of a predefined location of the image. For example, the reference data may include data of a particular pixel or data of a plurality of pixels constituting an image. The reference data may be data of a reference location from which the region growing is started. In this case, the reference data may include at least one of information regarding a location of a seed point of the region growing or a pattern of surrounding areas thereof, location data of the reference location, and image data of the reference location. Here, the image data of the reference location may include image data of the reference location alone. The image data of the reference location may also include data acquired using the image data of the reference location and image data of one or more neighboring locations. For example, the data acquired using the image data of the reference location and the image data of neighboring locations may be a mean value or a median between the image data of the reference location and that of the neighboring locations.

The reference data may or may not be changed during the region growing process. The reference data may be pre-defined before the region growing is started or may be selected by the user or the image processing apparatus during the region growing process. When the reference location is changed, the reference data may also be changed.

The reference location may constitute at least one location in the image. The reference location may be pre-defined before the region growing is started or may be selected by the user or the image processing apparatus during the region growing process. The reference location may be changed during the region growing process by the user or the image processing apparatus.

The image processing controller 200 may detect image data I using a calculated difference between the image data I and the reference data. For example, first, the image processing controller 200 calculates a difference between the image data I constituting the image and the reference data and determines whether the calculation result is less than a predefined threshold. When the calculation result is less than the predefined threshold, the image data I compared with the reference data is selected and detected. On the contrary, when the calculation result is greater than the predefined threshold, the image data I compared with the reference data is not detected. Accordingly, the image processing controller 200 may detect at least one portion of image data from among the image data I based on the comparison result. In this regard, as the predefined threshold decreases, image data identical or similar to the reference data may be detected. On the contrary, as the predefined threshold increases, image data different from the reference data may be detected by the image processing controller 200.

In addition, the image processing controller 200 may detect at least one portion (i.e. a portion less than whole) of image data from the image data I by a method commonly used in multiscale vessel enhancement filtering algorithm or multi-resolution vessel segmentation algorithm.

Hereinafter, the detection region now will be described in more detail. The image processing controller 200 compares image data of a predefined region, i.e., detection region, of the image with the reference data, and then detects at least one portion of image data from image data of the detection region based on the comparison result. The detection region indicates a predefined region of the image which is formed of an array of image data to be compared with the reference data. The detection region may be determined by a user selection or by pre-defined system setting. At least one detection region may be determined by at least one reference location.

More particularly, the image processing controller 200 may determine the location of at least one detection region according to the reference location and may determine at least another detection region in the image according to the determined location of the detection region. For example, the user may select the reference location using a reference location selection unit 101 or the pressure sensor 100. Then, the image processing controller 200 is configured to identify the location of the detection region based on the reference location selected by the user. When the detection region is determined in the image, the image processing controller 200 may determine a region within a predefined range as the detection region based on the determined location of the detection region. According to an embodiment, the detection region may be a portion of the image including all areas within a predefined distance from the reference location. According to another embodiment, the detection region may be a portion of the image including all areas within a square or rectangle formed around the reference location.

The detection region may be changed. In this case, the detection region may change as the reference location is changed. The image processing controller 200 may determine a first detection region using a first location of the image as a reference location, and detect at least one portion of image data from the determined first detection region. Then, when the reference location is changed to a second (different) location, the image processing controller 200 may update the reference data using the image data of the changed reference location. Thus, the reference data may vary during the image processing operation. Then, the image processing controller 200 may determine a second detection region using a second location different from the first location as a reference location and detect at least another portion of image data from the determined second detection region. The first and second portions may overlap or may not overlap. As a result, the image processing controller 200 may detect at least one portion of image data by moving the detection region. The detection region is changed or moved by changing or moving the reference location according to the descriptions given above. However, the detection region is not necessarily determined by changing or moving the reference location.

According to another embodiment, the image processing controller 200 may use only the pre-defined reference data although the reference location and the detection region are changed during image processing. For example, image data of an initially selected reference location, i.e., an initial reference location, may always be used as the reference data for comparison. In this case, the reference data may not be changed.

The size of the detection region may be determined according to the amount of pressure sensed by the pressure sensor 100 as described above. Particularly, the image processing controller 200 increases the range of the detection region as the amount of applied pressure sensed by the pressure sensor 100 increases and reduces the range of the detection region as the amount of pressure sensed by the pressure sensor 100 decreases. In this regard, the range of the detection region may arithmetically or geometrically increase according to the changed amount of applied pressure. According to another embodiment, the range of the detection region may be decreased proportionally or non-proportionally as pressure increases, and the range of the detection region may be increased as pressure decreases.

Meanwhile, when the range of the detection region is changed, resolution for detection of an image within the detection region may or may not be changed. For example, the number of pieces (i.e. portions) of image data may be reduced by decreasing resolution for detecting the image when a relatively wide range of the detection region is determined. On the contrary, a plurality of pieces (i.e. portions) of image data may be detected by increasing resolution for detecting the image when a relatively narrow range of the detection region is determined.

When the range of the detection region is determined based on the amount of pressure sensed by the pressure sensor 100, the image processing controller 200 compares the image data of the detection region having the determined range with the reference data and detects at least one portion of image data from the image data of the detection region based on the comparison result.

The image processing controller 200 may generate an image based on the detected at least one portion of image data. According to an embodiment, the image processing controller 200 may generate a plurality of images based on detected image data whenever the at least one portion of image data is detected. According to another embodiment, the image processing controller 200 may generate a plurality of images based on image data periodically detected at a predefined time interval. In this case, the predefined time interval may be determined by changeable settings pre-stored (i.e. pre-defined) in a system. The pre-stored settings may be changed by the user. In addition, the image processing controller 200 may generate an image based on the detected image data whenever the reference location is updated. The generated images may be stored in a storage device of the image processing apparatus. The storage device may include a semiconductor memory device or a disc storage device, but in any event, provides non-transitory storage.

The image processing controller 200 may also correct the previously-generated image based on the detected at least one portion of image data to generate a new image. For example, the image processing controller 200 may correct the previously- generated image by overlapping the detected at least one portion of image data and the previously generated image data. In this case, the image processing controller 200 may correct the previously-generated image by replacing the image data of the location corresponding to the detected at least one portion of image data among the previously generated image data with the detected at least one portion of image data. The correction of the image using the detected at least one portion of image data may be performed whenever the at least one portion of image data is detected, periodically performed at a predefined time interval, or performed whenever the reference location is changed, as described above.

The image generated or corrected by the image processing controller 200 may be displayed on the display unit 300. The display unit 300 may display a region-grown image generated by the image processing controller 200. The display unit 300 may display an image in which the region growing is being processed or completed. The display unit may periodically display an image in which the region growing is being processed or completed. The display unit 300 may display an image in which the region growing is being processed in real time. A status of the processing may be displayed.

When a plurality of images are acquired by region growing, the display unit 300 may display at least one of the images. In this case, the plurality of such acquired images by region growing may sequentially be acquired. The sequentially acquired images may be stored in a storage device of the image processing apparatus temporarily or non-temporarily. The display unit 300 may display the last stored image among the sequentially stored images or an image stored before the last stored image according to the user selection.

According to an embodiment, the display unit 300 may display a range of the detection region of image data that is one of the region growing parameters.

The display unit 300 may further display an indicator to display the sensed amount of pressure. The indicator may have various shapes or colors according to the amount of pressure. Moreover, the indicator may display the amount of pressure using numbers or images.

Meanwhile, the image processing apparatus may further include the reference location selection unit 101 as illustrated in FIG. 1. The user may select the reference location in the image using the reference location selection unit 101. The reference location selection unit 101, which comprises hardware such as integrated circuitry that may be configured for operation with machine executable code, may include an interface that can receive various input units connected to the image processing apparatus such as a keyboard, a mouse, a track pad, a track ball, and a tablet pad, wired or wirelessly, just to name a few non-limiting possibilities.

FIG. 3 is a block diagram illustrating an image processing apparatus according to another embodiment of the present invention. Referring now to FIG. 3, the image processing apparatus may include a touchscreen 102 and an image processing controller 200.

The touchscreen 102 receives an instruction according to the detection of touch (or near-touch) manipulation onto the touchscreen 102 and displays an image output from the image processing controller 200, or the like, to the user. According to an embodiment, the touchscreen 102 may sense pressure applied by the user via touch manipulation (as opposed to mere contact) and may also sense and measure an amount of the pressure. The touchscreen 102 may perform functions of the pressure sensor 100 as described above. For example, the touchscreen 102 may be a pressure-sensitive touchscreen that senses input pressure and can sense different amounts of input pressure. In addition, the touch screen 102 may include various other types of touchscreens capable of sensing the amount of pressure. The touchscreen 102 may measure the amount of pressure applied to the uppermost layer of the touchscreen 102 according to the touch manipulation using a finger of the user or a separate touch tool such as a touch pen and transmits to the image processing controller 200 a signal indicating the measured amount of applied pressure that was received image processing controller. The image processing controller 200 may determine region growing parameters based on the amount of applied pressure indicated as being received by the touchscreen 102.

The touchscreen 120 may also receive information regarding the reference location according to touch manipulation. In other words, when the user applies touch (or near touch) manipulation to a location of the touchscreen 102, the touchscreen 102 collects information regarding the location to which the touch is applied and transmits the information regarding the location to the image processing controller 200. The image processing controller 200 may receive information regarding the location to which the touch is applied and determine the location as a reference location. According to an embodiment, the reference location may be determined according to the location to which the user applies the touch manipulation or may be determined by settings of the system regardless of the touch manipulation of the user.

When the region growing parameters and the reference location are determined, the image processing controller 200 may perform region growing based on the region growing parameters.

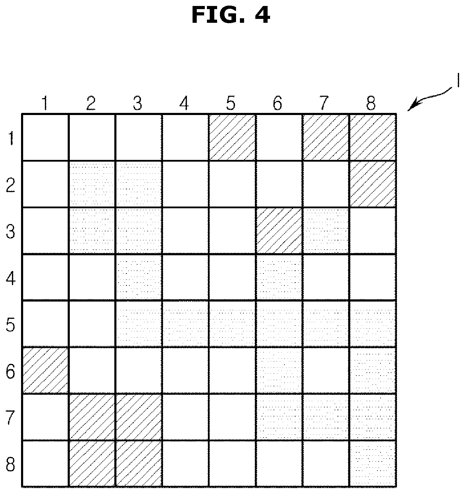

FIGS. 4 to 14 are diagrams illustrating exemplary operation of the image processing controller.

FIG. 4 illustrates an image I divided into a plurality of blocks. Each block of FIG. 4 may be a pixel of an image or a group of pixels. Meanwhile, although each block shown in FIG. 4 has a square shape, the block may also have a rectangular, hexagonal, equilateral triangular, circular shape or an irregular shape. In each block, image data corresponding to the particular block may exist. However, image data may not exist in some blocks. Image data in each of the blocks may be the same or different from one another.

Hereinafter, in descriptions of the image with reference to FIGS. 4 to 14, the blocks are respectively identified using numbers marked at top and left sides of the image. In other words, in FIG. 4, a block located at the top left corner is referred to as block 11 and is defined by number 1 at the left side of the block and number 1 at the top side thereof. A block on the bottom side of block 11 is referred to as block 21.

In addition, in FIGS. 4 through 14, it is considered that blocks having the same pattern, e.g., blocks 22 and 23 or blocks 17 and 18 (using the number system described above), have the same image data, unless otherwise stated. On the contrary, it is considered that bocks having different patterns, e.g., blocks 36 and block 37, have different image data, unless otherwise stated. Meanwhile, the image data may be, for example, row data or data processed by a predefined image processing method.

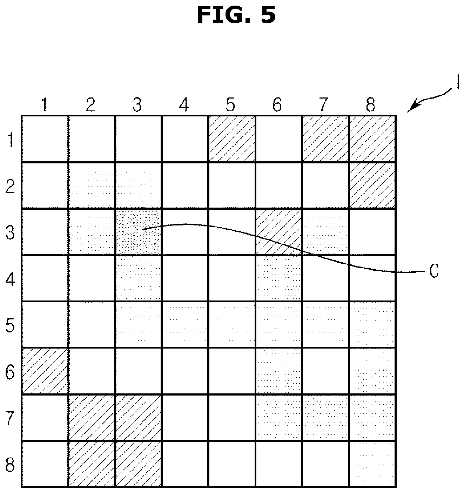

Hereinafter, the region growing will be described in more detail with reference to FIGS. 5 to 14. Referring now to FIG. 5, one of the blocks on the image I may be selected as a reference location "C". For example, block 33 may be selected as the reference location C as illustrated in FIG. 5. The reference location C may be determined by pre-defined settings of the system or by the user selection. When the reference location C is determined by the user selection, the user may select the reference location C via detection of the pressure sensor 100 such as a pressure-sensitive touchscreen 102. In addition, the reference location C may be selected using the separate reference location selection unit 101 (FIG. 1).

According to an embodiment, image data of the reference location C may be used as reference data to be compared with image data of another block. According to another embodiment, a mean value between image data of the reference location C and image data of neighboring locations of the reference location C may be used as the reference data. In addition, a median, a mode, a maximum value, or a minimum value between the image data of the reference location C and image data of the neighboring locations may also be used as the reference data.

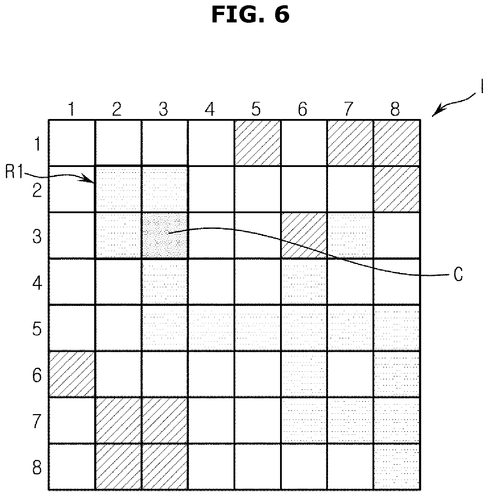

According to an embodiment, when the reference location C is selected as illustrated in FIG. 5, then as illustrated in FIG. 6 the neighboring locations of the reference location C may be determined as a detection region R1. This process may be performed by the image processing controller 200. Then, the image processing controller 200 compares image data of each of the blocks 22 to 44 of the detection region R1 with the reference data, for example, image data of the reference location (block 33). In this case, the image processing controller 200 calculates a difference between image data of each block of the detection region R1 with the reference data, determines whether the difference is less than a predefined threshold, and detects blocks from the detection region R1 or image data of the blocks when the difference is less than the predefined threshold.

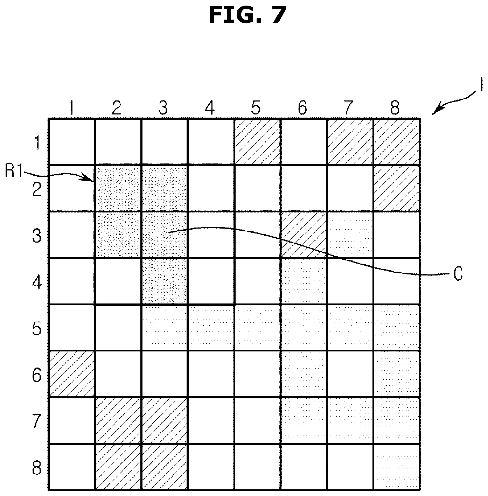

As a result, as illustrated in FIG. 7, blocks having image data identical or similar to the reference data (blocks 22, 23, 32, and 43) or image data thereof may be detected.

Meanwhile, the size of the detection region R1 may be determined by the amount of pressure applied to the pressure sensor 100 such as via the touchscreen 102.

For example, the size of the detection region R1 may be determined by reducing the size of the detection region R1 when a lower pressure is applied to the pressure sensor 100 and by increasing the size of the detection region when a higher pressure is applied to the pressure sensor 100.

However, the size of the detection region R1 may be determined in the opposite manner as described in the previous paragraph according to a system designer or settings by the user. For example, when the user applies a relatively low pressure to the pressure sensor 100, the detection region R1 may be determined as neighboring locations of the reference location C as illustrated in FIGS. 6 and 7.

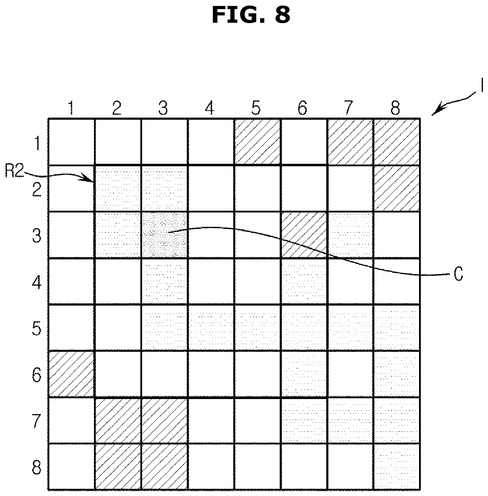

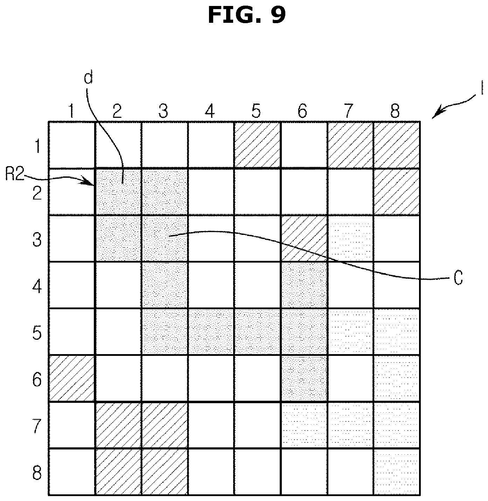

On the contrary, as shown in FIGs. 8 and 9, when the user applies a relatively high pressure to the pressure sensor 100, a wider detection region R2 may be determined. In this case, the display unit 300 may display the size of the detection region to allow the user to see the size. In this case, the detection region may be expressed using a frame partitioning the screen such as a circular or rectangular frame to distinguish the detection region from the other regions. Any other type of visual distinguishment may also be used to allow the user to see the size of the detection region.

As described above, when the detection region R2 is determined, the image processing controller 200 compares image data of each block in the detection region R2 (blocks 22 to 66) with the reference data, e.g., image data of the reference location C (block 33), and detects at least one portion of image data d as illustrated in FIG. 8. In this case, a difference between image data of each block of the detection region R2 and the reference data may also be used. Among image data of the detection region R2, image data different from the reference data, i.e., image data of the block (e.g., block 36) of the detection region R2 considerably different from the reference data may not be detected.

Upon comparison between FIGS. 7 and 9, image data detected in different detection regions R1 and R2 are different at least according to the size of the respective detection region. Since the sizes of the detection regions R1 and R2 are determined according to the amounts of pressure applied to the pressure sensor 100 as described above, the user may simply and efficiently control a displayed image.

According to an embodiment, as illustrated in FIGS. 7 and 9, after image data is detected from the detection regions R1 and R2, an image may be generated using the detected image data "d". In this case, the reference data C may also be used.

Meanwhile, according to an embodiment, the image processing controller 200 may detect image data from a detection region (first detection), and then change the detection region and further detect image data from the changed detection region (second detection).

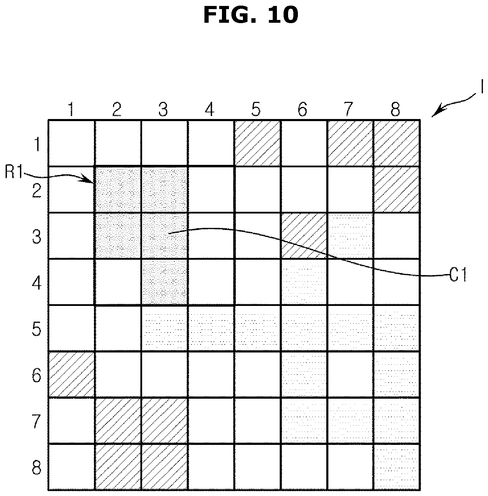

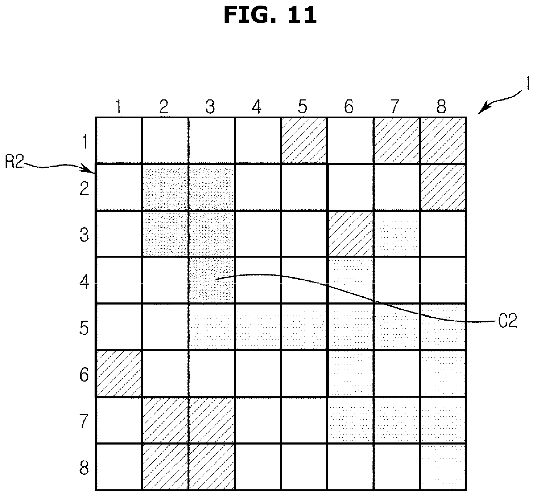

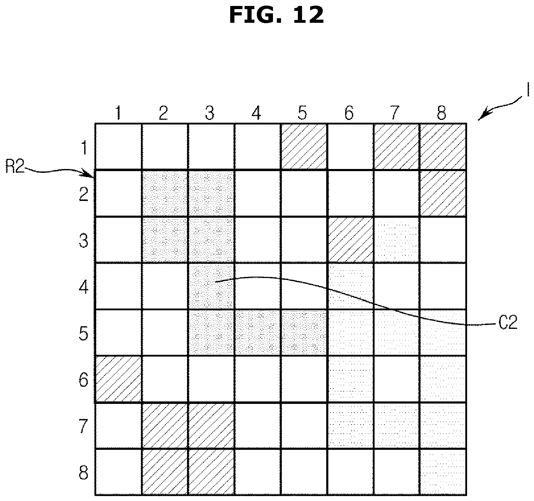

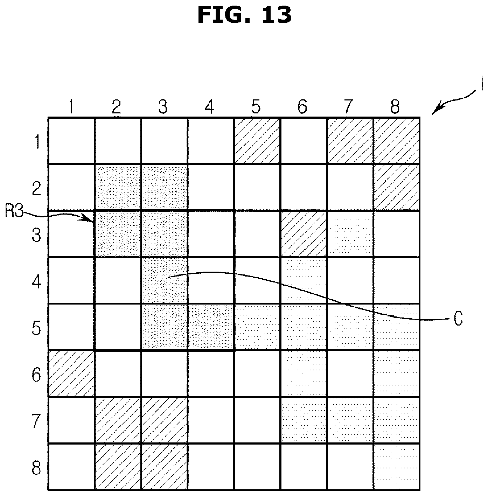

For example, as shown in FIG. 10. after image data is detected from a first detection region R1, different image data may further be detected from a second detection region R2 (in FIGs. 11 and FIG. 12) or a third detection region R3 (in FIG. 13) as illustrated in FIGS. 11, 12 and 13. In this case, the first detection region R1 and the second detection region R2 or the third detection region R3 may partially overlap each other. However, the sizes or locations thereof may be different from each other.

According to an embodiment shown in FIG. 11, when the location of the first detection region R1 is determined according to a first reference location C1, the image processing controller 200 may determine a second reference location C2 different from the first reference location C1 as illustrated in FIG. 10. According to an embodiment, the second reference location C may be determined as one block corresponding to image data detected from the first detection region R1. In this case, the image processing controller 200 may update the reference location as the second reference location C2 and determine the detection region using the updated second reference location C2.

In addition, the second reference location C2 may be determined by the user manipulation. For example, the user who selects the first reference location C1 by touching one portion of the touchscreen 102 may select the second reference location C2 by touching another portion of the touchscreen 102. In addition, after selecting the first reference location C1 by touching one portion of the touchscreen 102, the user may move a touch unit, (e.g., a stylus or a finger), to another portion of the touchscreen 102 while still being in a state of being in contact with the touchscreen 102, and stopping movement of the touch unit to select the second reference location C2. In addition, the image processing controller 200 may determine the size of the second detection region R2 according to the amount of pressure sensed by the pressure sensor 100. As a result, the image processing controller 200 may determine the second detection region R2 according to the determined second reference location C2 and the size of the second detection region R2. Then, as shown in FIG. 12, the image processing controller 200 may detect image data (blocks 53 to 55) from the determined second detection region R2 as illustrated in FIG. 12.

As described above, the image processing controller 200 may compare image data of each block of the second detection region R2 with the reference data, and then detect image data from the second detection region R2 based on the comparison results. As described above, in order to detect image data from the second detection region R2, a difference between image data of each block in the second detection region R2 and the reference data may be used. When image data of a block, e.g., block 61 of FIG. 12, does not satisfy detection conditions, the image processing controller 200 may not detect image data of block 61. According to an embodiment, reference data used to detect the image data of blocks 53 to 55 may be image data of the first reference location C1. According to another embodiment, the reference data may be a mean value between image data of the first reference location C1 and image data of neighboring locations thereof. According to another embodiment, the reference data used to detect the image data of blocks 53 to 55 may be image data of the second reference location C2 or a mean value between image data of the second reference location C2 and image data of neighboring locations thereof.

Meanwhile, as illustrated in FIGS. 12 and 13, after image data is detected from the first detection region R1, the second detection region R2 or the third detection region R3 having different sizes may be determined according to pressure applied to the pressure sensor 100 by the user. When the user applies a relatively high pressure to the pressure sensor 100, image data of blocks 53 to 55 may be additionally acquired from a wider region (second detection region R2). On the other hand, when the user applies a relatively low pressure to the pressure sensor 100, image data of blocks 53 and 54 may be acquired from a narrower region (third detection region R3).

According to an embodiment, images may respectively be generated using image data detected from respective detection operations, i.e., first detection and second detection. For example, the image processing controller 200 may generate an image based on the detected image data whenever each image data detection operation is completed. The image processing controller 200 may also generate an image based on the detected image data whenever the reference location is updated during each detection operation.

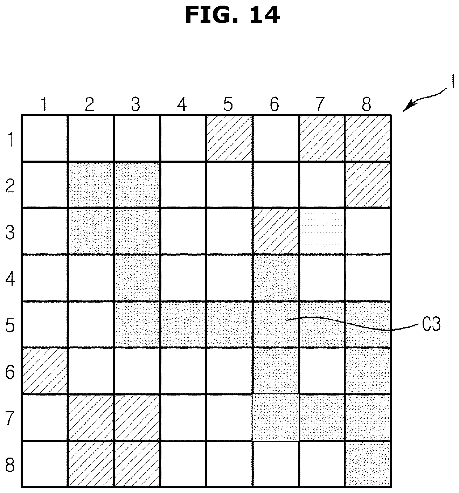

As a result, as illustrated in FIG. 14. image data satisfying uniform comparison conditions may be detected from one image by repeating image data detection operations in the same manner. As described above, when image data is detected using the difference between image data of each block and the reference data, and the reference data are the same or similar to each other, the same or similar image data may only be detected.

Meanwhile, since a plurality of portions of image data may be detected from a large detection region in each detection operation, the resultant image data may be obtained using a reduced number of operations. On the contrary, since a small number of portions of image data may be detected from a small detection region in each detection operation, the resultant image data may be obtained using an increased number of operations. Thus, when the user applies a higher pressure to the pressure sensor 100, the resultant image data may be obtained using a reduced number of operations. On the contrary, when the user applies a lower pressure to the pressure sensor 100, the resultant image data may be obtained using an increased number of operations. When an image is generated at each operation, the user may go over the process of acquiring the resultant image by applying a lower pressure to the pressure sensor 100.

The aforementioned image processing apparatus may be applied to radiation imaging apparatuses or magnetic resonance imaging apparatuses (not shown).

A radiation imaging apparatus may include a radiation emitter, a radiation detector, a pressure sensor, and an image processing controller. The radiation emitter may emit radiation having a predefined energy spectrum to an object. The radiation detector receives radiation having passed through the object and outputs an electric signal corresponding to the received radiation. Radiation emitted from the radiation emitter passes through the object. Since internal materials of the object have different attenuation rates, the amounts of radiation passing through the internal materials of the object vary. Based on this principle, the radiation imaging apparatus may examine an internal structure of the object. The pressure sensor may sense pressure applied by the user. The pressure sensor may be a touchscreen.

The image processing controller may generate a radiographic image based on the electric signal output from the radiation detector. The generated radiographic image may be displayed a display device directly mounted on the radiation imaging apparatus or a display device of a workstation connected to the radiation imaging apparatus. When the pressure sensor is a touchscreen, the radiographic image may be displayed on the touchscreen.

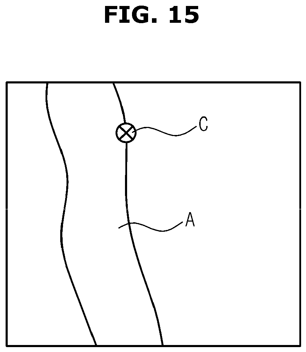

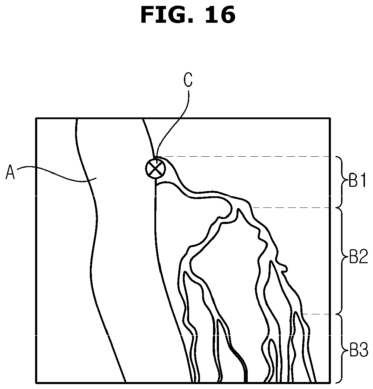

After identifying the radiographic image displayed on the display device, the user may perform additional image processing by applying predefined pressure to the pressure sensor. For example, as illustrated in FIGS. 15 and 16, only one portion of the internal structure displayed on the radiographic image may be displayed by region growing.

FIGS. 15 and 16 are diagrams illustrating region growing performed by using an image processing apparatus. FIG. 15 illustrates a radiographic image of a relatively thick blood vessel A such as artery or vein. Such radiographic image may be acquired by the radiation imaging apparatus, e.g., an angiography apparatus. The acquired radiographic image may be displayed on a display device of a workstation or a touchscreen.

The user may select one portion of the radiographic image as an initial reference location C using the pressure sensor or the touchscreen. The reference location may be selected using a separate reference location selection unit such as a mouse or a keyboard. In addition, the reference location may be selected according to system settings of the radiation imaging apparatus.

When the reference location C is selected, the image processing controller performs region growing from the reference location C as a starting point thereby generating an image of neighboring thinner blood vessels, e.g., capillary vessels, as illustrated in FIG. 16. Particularly, the image processing controller may determine region growing parameters based on the amount of sensed pressure and perform region growing based on the determined region growing parameters. Here, the region growing parameters may be the size of the detection region or the size of the object. The image processing controller may perform region growing by comparing image data of a detection region of the radiographic image determined by the sensed pressure with reference data, detecting at least one portion of image data from image data of the detection region based on the comparison result, and generating an image based on the detected image data. The image processing controller may use image data of the selected reference location C as the reference data. The reference data may be maintained or changed during the region growing process. In addition, the image processing controller may compare the image data of the detection region with the reference data by comparing a difference between the image data of the detection region and the reference data with a predefined threshold.

Meanwhile, while the image processing controller performs region growing, the user may adjust the size (sensitivity) of the detection region in the radiographic image by applying predefined pressure to the pressure sensor or touchscreen. As described above, the image processing controller may determine the size of the detection region of the radiographic image according to the amount of pressure applied to the pressure sensor.

Particularly, referring now to FIG. 16, in section B1 where a small number of thin capillary vessels are distributed, there is no need to perform region growing in a large detection region. The user may control the region growing to be performed in a small detection region by reducing the size of the detection region by reducing pressure applied to the pressure sensor or touchscreen. In this case, the image processing controller does not need to stop the region growing process. On the contrary, in section B2 having relatively thick capillary vessels or in section B3 having a number of capillary vessels, the region growing needs to be performed in a larger detection area. The user may control the region growing to be performed in a large detection region by increasing the size of the detection region by increasing pressure applied to the pressure sensor or touchscreen. In the same manner, the image processing controller does not stop the region growing process.

The user may appropriately adjust the size of the detection region depending on situation by changing pressure applied to the pressure sensor or touchscreen during the region growing process. As a result, an optimized image may be obtained.

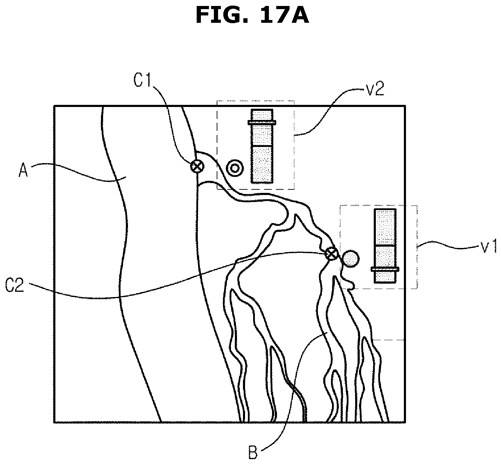

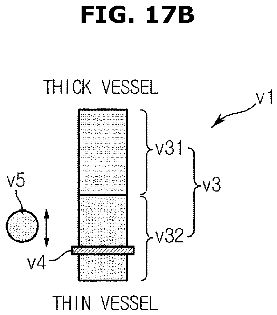

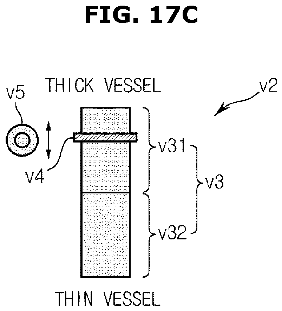

FIGS. 17A, 17B and FIG. 17C are diagrams for describing screens displaying indicators v1 and v2 which indicate pressure.

As illustrated in FIG. 17A, when a user applies an amount of pressure to the pressure sensor to perform region growing, the indicators v1 and v2, which indicate the applied pressure, may be displayed on a display device or a touch screen of a workstation. Accordingly, the user may easily recognize the amount of pressure.

The indicators v1 and v2 may respectively display different images according to the amount of pressure such that the user recognizes the amount of pressure, the size of the detection region, the size of the object, or the region growing rate. For example, the indicator may indicate the amount of pressure using upper and lower dimension bars v31 and v32, which indicate dimensions with respect to the amount of pressure, and an indication bar v4, which moves along the dimension bars v31 and v32 as a first indicator v3 illustrated in FIGS. 17A, 17B and17C. The upper dimension bar v31 may indicate a higher pressure, a wider detection region, or a larger or thicker object, and the lower dimension bar v32 may indicate a lower pressure, a narrower detection region, or a smaller or thinner object. The indicators v1 and v2 may indicate the amount of pressure by modifying a size of an inner circle as a second indicator v5 illustrated in FIGS. 17B and 17C. When there is no inner circle (as illustrated in FIG. 17B), the second indicator v5 may indicate no pressure or a smaller pressure than a first indicator. In this case, a radiation imaging apparatus may perform region growing by detecting image data in a narrower range of detection region. Alternatively, the radiation imaging apparatus may perform region growing while detecting a smaller or thinner object, e.g., narrower blood vessels such as capillary. As illustrated in FIG. 17C, the second indicator v5 may indicator a greater amount of pressure using a larger inner circle. In this case, the radiation imaging apparatus may perform region growing by detecting image data in a wider range of detection region. Alternatively, the radiation imaging apparatus may perform region growing while detecting a larger or thicker object, e.g., wider blood vessels such as artery or vein. The display device or the touch screen may include any one of the first indicator v3, or the second indicator v5, or both of the first and second indicators v3 and v5.

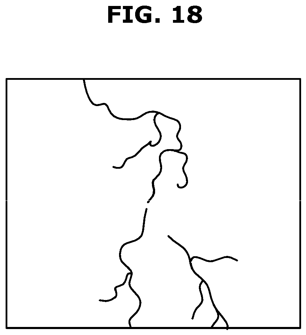

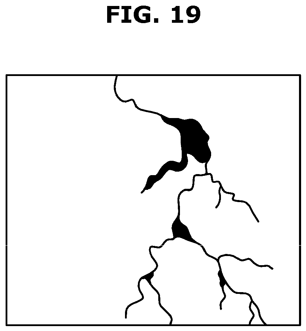

FIGS. 18 and 19 are diagrams for describing screens displayed by an image processing apparatus based on a threshold change. FIG. 18 is an image generated based on image data detected using a low threshold, and FIG. 19 is an image generated based on image data detected using a high threshold. The image processing controller may use a predefined threshold in comparison between the image data of the detection region and the reference data as described above. Particularly, the image data of the detection region may be compared with the reference data by calculating a difference between the image data of the detection region and the reference data and comparing the calculated difference with the predefined threshold.

When the calculated difference is less than the predefined threshold, the image data of the detection region may be detected. In this case, when a low threshold is used, image data of the detection region identical or similar to the reference data may only be detected as illustrated in FIG. 18. On the contrary, when a high threshold is used, image data different from the reference data may also be detected. Thus, more image data may be detected as illustrated in FIG. 19 than those of FIG. 18.

The radiation imaging apparatus as described above may be a digital radiography (DR) apparatus, a fluoroscopy apparatus, an electrocardiography apparatus, a computed tomography (CT) scanner, and a mammography system. Any other imaging apparatuses using radiation may also be used as the radiation imaging apparatus described above.

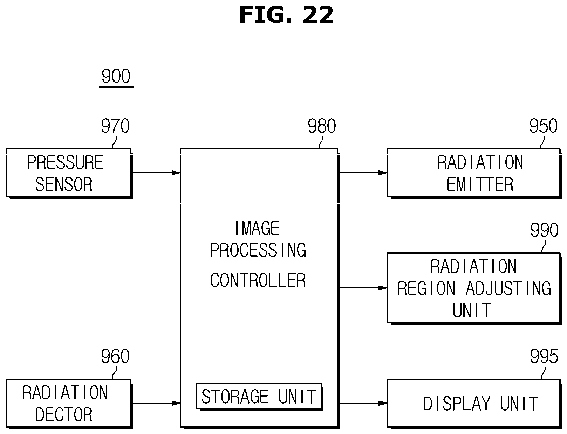

More particularly, in accordance with FIG. 22, an exemplary radiation apparatus 900 includes a radiation emitter 950 to emit radiation to an object. Radiation detector 960 is configured to receive radiation passed through the object and the radiation detector 960 outputs an electrical signal corresponding to the received radiation. A pressure sensor 970 senses an amount of applied pressure of a touch event. An image processing controller 980 generates a radiographic image based on the received electric signal by determining region growing parameters based on the sensed amount of applied pressure of the touch event, and performing a region growing operation from at least one reference location within the radiographic image based on the determined region growing parameters. Radiation Region adjusting unit 990 adjusts the amount of radiation for a particular region. Display unit 995 displays the generated radiographic image.

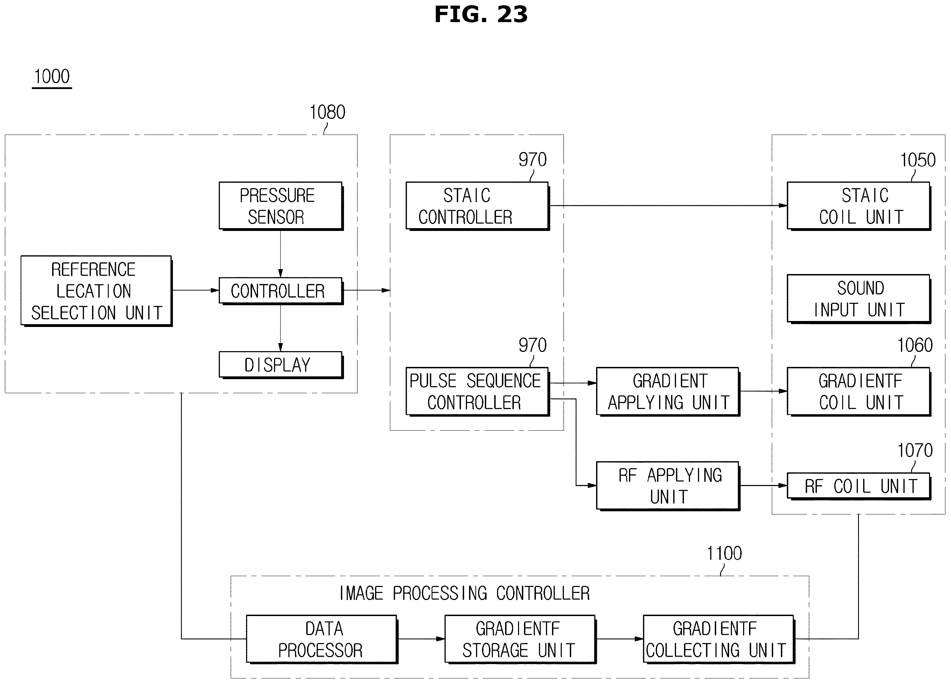

Meanwhile, as shown in FIG. 23, the magnetic resonance imaging apparatus 1000 may include a static magnetic field coil unit 1050, a gradient magnetic field coil unit 1060, a radio frequency (RF) coil unit 1070, a pressure sensor 1080, and an image processing controller 1100. The static magnetic field coil unit 1050 may form a static magnetic field with respect to an object, and the gradient magnetic field coil unit 1060 may form a gradient magnetic field with respect to the object. The RF coil unit 1070 applies electromagnetic waves to the object positioned in the static magnetic field and the gradient magnetic field to induce magnetic resonance in the object and receives magnetic resonance signals generated in accordance with the magnetic resonance. The RF coil unit 1070 may include a transmission coil to induce magnetic resonance and a reception coil to receive magnetic resonance signals. According to another example, the RF coil unit 1070 may include a transmission/reception coil capable of inducing magnetic resonance and receiving magnetic resonance signals. The pressure sensor may sense pressure applied by the user.

The pressure sensor may be a touchscreen, and more particularly the touchscreen of a manipulation console. The image processing controller 1000 may generate a magnetic resonance image based on the magnetic resonance signals. In the same manner as the radiation imaging apparatus, the magnetic resonance image may be displayed on a display device of a workstation or a touchscreen. The image processing controller 1100 may also perform a region growing process. The image processing controller 1100 may also determine region growing parameters based on the sensed amount of pressure, and may perform region growing from at least one reference location in the radiological image based on the determined region growing parameters.

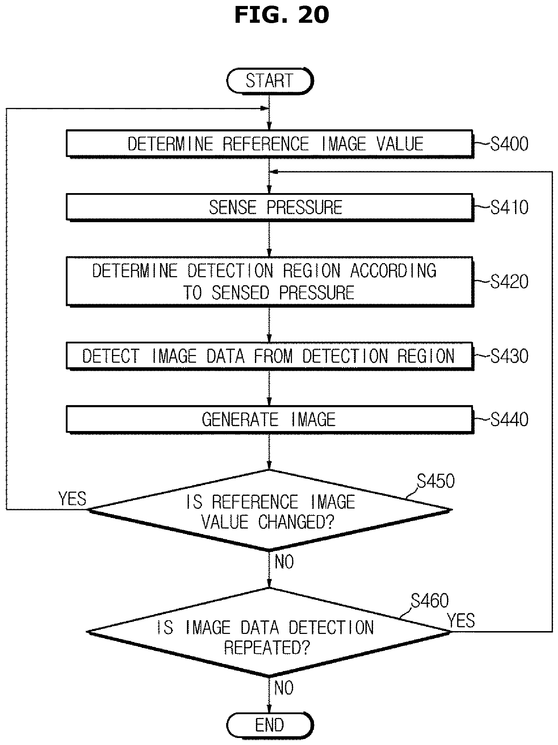

Hereinafter, in accordance with FIG. 20, a method of displaying an image will be described. FIG. 20 is a flowchart illustrating a method of displaying an image according to an embodiment of the present invention.

According to the method of displaying an image as illustrated in FIG. 20, at (S400) reference data. The reference data may be selected by a user or determined according to system settings. Image data of a reference location determined by the user of the system settings may be used as the reference data. In this regard, the reference location may indicate a predefined position in the image. The region growing may be started from the reference location.

With reference to (S410), when the reference data is determined, the pressure may be sensed, the pressure may be sensed by a pressure sensing unit or a touchscreen. The user applies pressure to the pressure sensing unit or touchscreen using a finger or a touch pen, and the pressure sensing unit or the touchscreen may sense the amount of pressure applied by the user.

At (S420), region growing parameters may be determined according to the sensed pressure. The region growing parameters may include at least one of a range of the detection region of image data, a region growing rate, and a size of the object. According to an embodiment, as the sensed pressure increases, at least one of the size of the detection region of the image data, the region growing rate, and the size of the object may increase. On the other hand, as the sensed pressure decreases, at least one of the size of the detection region of the image data, the region growing rate, and the size of the object may decrease.

At (S430), when the detection region is determined, image data may be detected from the detection region to perform region growing. Here, image data may be detected from the detection region based on the comparison result with the reference data determined in operation S400.

Accordingly, at (S440), when the image data is detected, an image is generated based on the detected image data.

At (S450), there is a determination whether the reference data has changed. For example, when the reference data is determined according to the reference location, change of the reference location may result in change of the reference data. However, the reference data may not be changed although the reference location is changed. When the reference data is changed, the previous reference data is updated using the changed reference data to determine a new reference data (S400), and operations S410 to S440 may be repeated.

When the reference data is not changed, at (S460), the detection of the image data may be repeatedly performed. In other words, when image data is repeatedly detected after detecting image data from the detection region, operations S410 to S440 may be repeated. In this case, the sensed amount of pressure may be changed, and accordingly, the size of the detection region may also be changed. The location of the detection region may also be changed. The change of the location of the detection region may be performed based on the change of the reference location. The change of the reference location may be performed according to the system settings or the user selection.

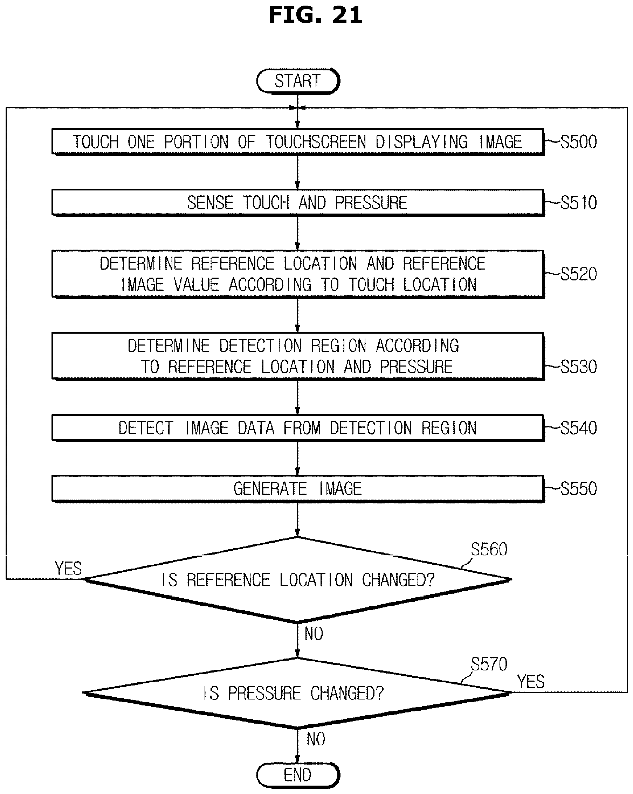

Hereinafter, a method of performing region growing using a touchscreen will be described with reference to FIG. 21. FIG. 21 is a flowchart illustrating a method of displaying an image according to another embodiment of the present invention.

Referring now to FIG. 21, at (S500) a user may touch one portion of a touchscreen displaying an image using a finger or a separate touch pen. In this regard, the user may apply predefined pressure to the touchscreen while touching the portion of the touchscreen.

Then at (S510), the touchscreen may sense the touch and pressure applied by the user.

At (S520), the image processing apparatus may determine a reference location based on the touch point and determine image data of the reference location as an initial reference data.

At (S530), the image processing apparatus may determine the region growing parameters according to the reference location and the sensed amount of pressure. The region growing parameters may include at least one of a range of the detection region of the image data, a region growing rate, and a size of the object. In this case, the range of the detection region of the image data, the region growing rate, and the size of the object may be proportional or inverse proportional to the sensed amount of pressure.

Then at (S540), the region growing may be performed by comparing image data of the detection region with the reference data, and detecting at least one portion of image data from the image data of the detection region based on the comparison result.

At (S550), when image data is detected from the detection region, an image may be generated using the detected image data. In this case, the reference data of the reference location may be used to generate the image.

Operations S500 to S550 as described above may be repeated. The repetition of operations S500 to S550 may be performed when the reference location is changed (S560) or the pressure is changed (S570).

The change of the reference location may be determined by the user selection or system settings. The user may change the reference location by changing the touch point on the touchscreen. In this regard, the user may change the reference location by detaching the previous touch from the touchscreen and touching another point, or may change the reference location by sliding the finger or the touch pen in a state of being in contact with the touchscreen. Meanwhile, the changed reference location may be one point within the detection region. The change of the pressure may be adjusted by increasing or decreasing pressure applied to the touchscreen while the user is maintaining the touch.

As is apparent from the above description, the user may simply and efficiently control a displayed image according to pressure change through the method of displaying an image, the image processing apparatus, the radiation imaging apparatus, and the magnetic resonance imaging apparatus, and thus convenience of controlling the image display may be improved.

In addition, a user may acquire a region-grown image by simply controlling the region growing.

Furthermore, according to the method of displaying an image, the image processing apparatus, the radiation imaging apparatus, and the magnetic resonance imaging apparatus, a user may perform region growing without stopping the region growing process in order to adjust sensitivity according to the range of the region. Accordingly, the user may change sensitivity according to the range of the region for convenience or necessity of the user.

The above-described methods according to the present invention can be implemented in hardware, firmware or as software or computer code that configures hardware for operation, and is stored on a non-transitory machine readable medium such as a CD ROM, DVD, RAM, a floppy disk, a hard disk, or a magneto-optical disk, such as a floptical disk or computer code downloaded over a network originally stored on a remote recording medium or a non-transitory machine readable medium and stored on a local non-transitory recording medium, so that the methods described herein can be loaded into hardware such as a general purpose computer, or a special processor or in programmable or dedicated hardware, such as an ASIC or FPGA. As would be understood in the art, the computer, the processor, microprocessor controller or the programmable hardware include memory components, e.g., RAM, ROM, Flash, etc. that may store or receive software or computer code that when accessed and executed by the computer, processor or hardware implement the processing methods described herein. In addition, it would be recognized that when a general purpose computer accesses code for implementing the processing shown herein, the execution of the code transforms the general purpose computer into a special purpose computer for executing the processing shown herein. In addition, an artisan understands and appreciates that a "processor" or "microprocessor" comprise hardware in the claimed invention.

When a unit, module, processor, microprocessor, controller, etc., includes machine executable code, it is to be understood that a non-transitory machine readable medium contains the as a processor or controller for execution and configures the hardware (such as, for example, the processor or controller) for operation.

## Claims

1. An image processing apparatus comprising:
a pressure sensor (100) to sense an amount of pressure associated with a touch event; and
an image processing controller (200) configured to generate at least one image of an object by determining a reference location of the touch event and determining region growing parameters based on the sensed amount of pressure received from the pressure sensor, and to perform a region growing operation from the reference location based on the region growing parameters, and
wherein the region growing parameters comprise a range of a detection region of image data, wherein the detection region indicates a predefined region of the image which is formed of an array of image data to be compared with the reference data of the reference location; and
wherein the image processing controller (200) is configured to increase the range of the detection region of image data as the sensed amount of pressure associated with the touch event increases and to decrease the range of the detection region of image data as the sensed amount of pressure associated with the touch event decreases.

2. The image processing apparatus according to claim 1, wherein the region growing parameters further comprise at least one parameter selected from the group consisting of a region growing rate, and a size of the object.

3. The image processing apparatus according to claim 1, wherein the image processing controller (200) increases at least one parameter selected from the group consisting of the region growing rate, and the size of the object as the sensed amount of pressure of the touch event increases.

4. The image processing apparatus according to claim 1, wherein the image processing controller (200) performs the region growing by comparing data of at least one location within the detection region with the reference data of the reference location.

5. The image processing apparatus according to claim 4, wherein the reference data comprises at least one item selected from the group consisting of image data of the reference location, location data of the reference location, and a shape of neighboring locations of the reference location.

6. The image processing apparatus according to claim 5, wherein the image data of the reference location (c) comprises values acquired using both the image data of the reference location (c) and image data of the neighboring locations of the reference location (c).

7. The image processing apparatus according to claim 4, wherein the image processing controller (200) compares data of the detection region with the reference data of the reference location, and detects data based on the comparison result.

8. The image processing apparatus according to claim 7, wherein the image processing controller (200) generates at least one image based on detected image data when detection of image data in the detection region is complete.

9. The image processing apparatus according to claim 1, wherein the image processing controller (200) generates a plurality of images of the object by performing the region growing operation a plurality of times from different reference locations.

10. The image processing apparatus according to claim 9, further comprising a storage unit configured to sequentially store the plurality of images in an order in which the images are generated.

11. The image processing apparatus according to claim 1, further comprising a display unit (300) to display the at least one image output by the image processing controller.

12. The image processing apparatus according to claim 11, wherein the display unit (300) displays a range of a detection region of image data, displays at least one image from among a plurality of sequentially stored images, or displays an indicator indicating an amount of pressure sensed by the pressure sensor.

13. The image processing apparatus according to claim 1, wherein the pressure sensor (100) comprises a touchscreen (102) to sense the amount of pressure applied to the touchscreen (102) during the touch event and display an indicator indicating the sensed amount of pressure.

14. The image processing apparatus according to claim 1, wherein the pressure sensor (100) senses pressure of the touch event associated with a particular instruction to select the reference location.

15. A method of displaying an image, the method comprising:
sensing by a pressure sensor (100) an amount of applied pressure of a touch event;
receiving by an image processing controller (200) an output signal indicating the amount of applied pressure sensed by the pressure sensor;
determining by the image processing controller (200) a reference location of the touch event;
determining by the image processing controller parameters associated with the reference location of the touch event and determining region growing parameters based on the sensed amount of applied pressure; and
performing by the image processing controller (200) a region growing operation from the determined reference location using the determined region growing parameters, and
wherein the region growing parameters comprise a range of a detection region of image data, wherein the detection region indicates a predefined region of the image which is formed of an array of image data to be compared with the reference data of the reference location; and
wherein the image processing controller (200) is configured to increase the range of the detection region of image data as the sensed amount of pressure associated with the touch event increases and to decrease the range of the detection region of image data as the sensed amount of pressure associated with the touch event decreases.

## Patentansprüche

1. Bildverarbeitungsvorrichtung, die umfasst:
einen Drucksensor (100), um eine Menge an Druck zu erfassen, die mit einem Berührungsereignis verknüpft ist; und
eine Bildverarbeitungssteuerung (200), die dazu konfiguriert ist, durch Bestimmen einer Referenzposition des Berührungsereignisses und Bestimmen von Bereichswachstumsparametern basierend auf dem Empfang der von dem Drucksensor erfassten Menge an Druck wenigstens ein Bild eines Objekts zu erzeugen, und dazu, basierend auf den Bereichswachstumsparametern einen Bereichswachstumsvorgang von der Referenzposition durchzuführen, und
wobei die Bereichswachstumsparameter eine Ausmaß eines Erfassungsbereichs von Bilddaten umfassen, wobei der Erfassungsbereich einen vordefinierten Bereich des Bildes anzeigt, der aus einer Anordnung von Bilddaten, die mit den Referenzdaten der Referenzposition verglichen werden sollen, gebildet ist; und
wobei die Bildverarbeitungssteuerung (200) dazu konfiguriert ist, die Ausmaß des Erfassungsbereichs von Bilddaten zu erhöhen, wenn die erfasste Menge an Druck, die mit dem Berührungsereignis verknüpft ist, sich erhöht, und dazu, die Ausmaß des Erfassungsbereichs von Bilddaten zu verringern, wenn die erfasste Menge an Druck, die mit dem Berührungsereignis verknüpft ist, sich verringert.

2. Bildverarbeitungsvorrichtung nach Anspruch 1, wobei die Bereichswachstumsparameter weiterhin wenigstens einen Parameter umfassen, der aus der Gruppe bestehend aus einer Bereichswachstumsrate und einer Größe des Objekts ausgewählt wird.

3. Bildverarbeitungsvorrichtung nach Anspruch 1, wobei die Bildverarbeitungssteuerung (200) wenigstens einen Parameter, der aus der Gruppe bestehend aus der Bereichswachstumsrate und der Größe des Objekts ausgewählt wird, erhöht, wenn die erfasste Menge an Druck des Berührungsereignisses sich erhöht.

4. Bildverarbeitungsvorrichtung nach Anspruch 1, wobei die Bildverarbeitungssteuerung (200) das Bereichswachstum durchführt mittels eines Vergleichs der Daten von wenigstens einer Position innerhalb des Erfassungsbereichs mit den Referenzdaten der Referenzposition.

5. Bildverarbeitungsvorrichtung nach Anspruch 4, wobei die Referenzdaten wenigstens ein Element umfassen, das aus der Gruppe bestehend aus Bilddaten der Referenzposition, Positionsdaten der Referenzposition und Form von benachbarten Positionen der Referenzposition ausgewählt ist.

6. Bildverarbeitungsvorrichtung nach Anspruch 5, wobei die Bilddaten der Referenzposition (c) Werte umfassen, die unter Verwendung sowohl der Bilddaten der Referenzposition (c) als auch von Bilddaten der benachbarten Positionen der Referenzposition (c) erhalten werden.

7. Bildverarbeitungsvorrichtung nach Anspruch 4, wobei die Bildverarbeitungssteuerung (200) Daten des Erfassungsbereichs mit den Referenzdaten der Referenzposition vergleicht und Daten basierend auf dem Ergebnis des Vergleichs erfasst.

8. Bildverarbeitungsvorrichtung nach Anspruch 7, wobei die Bildverarbeitungssteuerung (200) basierend auf erfassten Bilddaten wenigstens ein Bild erzeugt, wenn die Erfassung von Bilddaten in dem Erfassungsbereich vollendet ist.

9. Bildverarbeitungsvorrichtung nach Anspruch 1, wobei die Bildverarbeitungssteuerung (200) mittels Durchführen des Bereichswachstumsvorgangs mehrere Male von unterschiedlichen Referenzpositionen eine Mehrzahl von Bildern des Objekts erzeugt.

10. Bildverarbeitungsvorrichtung nach Anspruch 9, die weiterhin eine Speichereinheit umfasst, welche dazu konfiguriert ist, die Mehrzahl von Bildern in der Reihenfolge, in der sie erzeugt worden sind, nacheinander zu speichern.

11. Bildverarbeitungsvorrichtung nach Anspruch 1, die weiterhin eine Anzeigeeinheit (300) umfasst, um das wenigstens eine von der Bildverarbeitungssteuerung ausgegebene Bild anzuzeigen.

12. Bildverarbeitungsvorrichtung nach Anspruch 11, wobei die Anzeigeeinheit (300) ein Ausmaß eines Erfassungsbereichs von Bilddaten anzeigt, wenigstens ein Bild aus einer Mehrzahl von nacheinander gespeicherten Bildern anzeigt, oder einen Anzeiger anzeigt, der eine Menge an von dem Drucksensor erfasstem Druck angibt.

13. Bildverarbeitungsvorrichtung nach Anspruch 1, wobei der Drucksensor (100) einen Berührungsbildschirm (102) umfasst, um die Menge an während des Berührungsereignisses auf den Berührungsbildschirm (102) ausgeübtem Druck zu erfassen und einen Anzeiger anzuzeigen, der die erfasste Menge an Druck angibt.

14. Bildverarbeitungsvorrichtung nach Anspruch 1, wobei der Drucksensor (100) den Druck des mit einer bestimmten Anweisung zur Auswahl der Referenzposition verknüpften Berührungsereignisses erfasst.

15. Verfahren zum Anzeigen eines Bildes, wobei das Verfahren umfasst:
Erfassen, durch einen Drucksensor (100), einer Menge an ausgeübtem Druck, die mit einem Berührungsereignis verknüpft ist;
Empfangen, an einer Bildverarbeitungssteuerung (200), eines Ausgabesignals, das die Menge an von dem Drucksensor erfasstem ausgeübtem Druck angibt;
Bestimmen einer Referenzposition des Berührungsereignisses durch die Bildverarbeitungssteuerung (200);
Bestimmen, durch die Bildverarbeitungssteuerung, von Parametern, die mit der Referenzposition des Berührungsereignisses verknüpft sind, und Bestimmen von Bereichswachstumsparametern basierend auf der erfassten Menge an ausgeübtem Druck; und
Durchführen, durch die Bildverarbeitungssteuerung (200), eines Bereichswachstumsvorgangs von der bestimmten Referenzposition unter Verwendung der bestimmten Bereichswachstumsparameter,
wobei die Bereichswachstumsparameter ein Ausmaß eines Erfassungsbereichs von Bilddaten umfassen, wobei der Erfassungsbereich einen vordefinierten Bereich des Bildes anzeigt, der aus einer Anordnung von Bilddaten, die mit den Referenzdaten der Referenzposition verglichen werden sollen, gebildet ist; und
wobei die Bildverarbeitungssteuerung (200) dazu konfiguriert ist, das Ausmaß des Erfassungsbereichs von Bilddaten zu erhöhen, wenn die erfasste Menge an Druck, die mit dem Berührungsereignis verknüpft ist, sich erhöht, und dazu, das Ausmaß des Erfassungsbereichs von Bilddaten zu verringern, wenn die erfasste Menge an Druck, die mit dem Berührungsereignis verknüpft ist, sich verringert.

## Revendications

1. Dispositif de traitement d'image comprenant:
un détecteur de pression (100) destiné à détecter une quantité de pression associée à un événement tactile, et
un organe de commande de traitement d'image (200) conçu pour générer au moins une image d'un objet en déterminant un emplacement de référence de l'événement tactile et en déterminant des paramètres d'élargissement de région en fonction de la quantité de pression détectée reçue en provenance du détecteur de pression, et pour réaliser une opération d'élargissement de région à partir de l'emplacement de référence en fonction des paramètres d'élargissement de région; dans lequel
les paramètres d'élargissement de région comprennent l'étendue d'une région de détection de données d'image, ladite région de détection indiquant une région prédéfinie de l'image constituée d'un ensemble de données d'image destinées à être comparées aux données de référence de l'emplacement de référence, et
l'organe de commande de traitement d'image (200) est conçu pour augmenter l'étendue de la région de détection des données d'image tandis que la quantité de pression détectée associée à l'événement tactile augmente et pour diminuer l'étendue de la région de détection des données d'image tandis que la quantité de pression détectée associée à l'événement tactile diminue.

2. Dispositif de traitement d'image selon la revendication 1, dans lequel les paramètres d'élargissement de région comprennent en outre au moins un paramètre choisi dans le groupe constitué par le taux d'élargissement de la région et la taille de l'objet.

3. Dispositif de traitement d'image selon la revendication 1, dans lequel l'organe de commande de traitement d'image (200) augmente au moins un paramètre choisi dans le groupe constitué par le taux d'élargissement de la région et la taille de l'objet tandis que la quantité de pression détectée de l'événement tactile augmente.

4. Dispositif de traitement d'image selon la revendication 1, dans lequel l'organe de commande de traitement d'image (200) réalise l'élargissement de la région en comparant les données d'au moins un emplacement au sein de la région de détection aux données de référence de l'emplacement de référence.

5. Dispositif de traitement d'image selon la revendication 4, dans lequel les données de référence comprennent au moins un élément choisi dans le groupe constitué par les données d'image de l'emplacement de référence, les données de l'emplacement de référence et la forme des emplacements voisins de l'emplacement de référence.

6. Dispositif de traitement d'image selon la revendication 5, dans lequel les données d'image de l'emplacement de référence (c) comprennent des valeurs acquises au moyen des données d'image de l'emplacement de référence (c) et des données d'image des emplacements voisins de l'emplacement de référence (c).

7. Dispositif de traitement d'image selon la revendication 4, dans lequel l'organe de commande de traitement d'image (200) compare les données de la région de détection aux données de référence de l'emplacement de référence, et détecte des données en fonction du résultat de la comparaison.

8. Dispositif de traitement d'image selon la revendication 7, dans lequel l'organe de commande de traitement d'image (200) génère au moins une image en fonction des données d'image détectées lorsque la détection des données d'image dans la région de détection est terminée.

9. Dispositif de traitement d'image selon la revendication 1, dans lequel l'organe de commande de traitement d'image (200) génère une pluralité d'images de l'objet en réalisant l'opération d'élargissement de région une pluralité de fois à partir de différents emplacements de référence.

10. Dispositif de traitement d'image selon la revendication 9, comprenant en outre une unité de stockage conçue pour stocker successivement la pluralité d'images dans l'ordre dans lequel les images sont générées.

11. Dispositif de traitement d'image selon la revendication 1, comprenant en outre une unité d'affichage (300) destinée à afficher l'au moins une image émise par l'organe de commande de traitement d'image.

12. Dispositif de traitement d'image selon la revendication 11, dans lequel l'unité d'affichage (300) affiche l'étendue d'une région de détection de données d'image, affiche au moins une image parmi une pluralité d'images successivement stockées, ou affiche un indicateur indiquant une quantité de pression détectée par le détecteur de pression.

13. Dispositif de traitement d'image selon la revendication 1, dans lequel le détecteur de pression (100) comprend un écran tactile (102) pour détecter la quantité de pression appliquée à l'écran tactile (102) au cours de l'événement tactile et pour afficher un indicateur indiquant la quantité de pression détectée.

14. Dispositif de traitement d'image selon la revendication 1, dans lequel le détecteur de pression (100) détecte la pression de l'événement tactile associé à une instruction particulière visant à choisir l'emplacement de référence.

15. Procédé d'affichage d'une image, le procédé comprenant:
la détection, par un détecteur de pression (100), d'une quantité de pression appliquée relative à un événement tactile,
la réception, par un organe de commande de traitement d'image (200), d'un signal de sortie indiquant la quantité de pression appliquée détectée par le détecteur de pression,
la détermination, par l'organe de commande de traitement d'image (200), d'un emplacement de référence de l'événement tactile,
la détermination, par l'organe de commande de traitement d'image, de paramètres associés à l'emplacement de référence de l'événement tactile, et la détermination de paramètres d'élargissement de région en fonction de la quantité de pression appliquée détectée, et
la réalisation, par l'organe de commande de traitement d'image (200), d'une opération d'élargissement de région à partir de l'emplacement de référence déterminé, au moyen des paramètres d'élargissement de région déterminés ; dans lequel
les paramètres d'élargissement de région comprennent l'étendue d'une région de détection de données d'image, ladite région de détection indiquant une région prédéfinie de l'image constituée d'un ensemble de données d'image destinées à être comparées aux données de référence de l'emplacement de référence, et
l'organe de commande de traitement d'image (200) est conçu pour augmenter l'étendue de la région de détection des données d'image tandis que la quantité de pression détectée associée à l'événement tactile augmente et pour diminuer l'étendue de la région de détection des données d'image tandis que la quantité de pression détectée associée à l'événement tactile diminue.
